# EUROPEAN PATENT SPECIFICATION

(11) **EP 3 868 870 B1**
(45) Date of publication and mention of the grant of the patent: **03.12.2025**
(21) Application number: 19874512.7
(22) Date of filing: 11.10.2019
(51) Int. Cl.: C12N 5/074, C12N 5/02

(54) **METHOD FOR PRODUCING STEM/PRECURSOR CELLS, BY USING LOW MOLECULAR WEIGHT COMPOUND, FROM CELLS DERIVED FROM ENDODERMAL TISSUE OR ORGAN**
VERFAHREN ZUR HERSTELLUNG STAMM-/VORLÄUFERZELLEN UNTER VERWENDUNG NIEDERMOLEKULARER VERBINDUNGEN AUS ZELLEN, DIE AUS EINEM ENDODERMALEN GEWEBE ODER ORGAN STAMMEN
PROCÉDÉ DE PRODUCTION DE CELLULES SOUCHES/PRÉCURSEURS, À L'AIDE D'UN COMPOSÉ DE FAIBLE POIDS MOLÉCULAIRE, À PARTIR DE CELLULES DÉRIVÉES DE TISSU OU D'ORGANE ENDODERMIQUE

(30) Priority: 15.10.2018 JP 2018194567
(43) Date of publication of application: 25.08.2021
(73) Proprietor: Evia Life Sciences Inc., Seattle WA 98109 (US)
(72) Inventor: OCHIYA, Takahiro, Tokyo 153-0042 (JP); MATSUZAKI, Juntaro, Tokyo 153-0042 (JP); ENOMOTO, Hideharu, Tokyo 153-0042 (JP); SHINOHARA, Masumi, Tokyo 153-0042 (JP)
(74) Representative: Potter Clarkson
(86) International application number: PCT/JP2019/041234
(87) International publication number: WO 2020/080550

(56) References cited:
- EP-A1- 3 031 905
- EP-A1- 3 178 924
- WO-A1-2007/039986
- WO-A1-2011/047300
- WO-A1-2013/163739
- WO-A1-2013/174794
- WO-A1-2014/044646
- WO-A1-2014/201167
- WO-A1-2016/021734
- WO-A1-2017/019702
- WO-A1-2017/119512
- WO-A1-2017/177163
- WO-A1-2018/079714
- WO-A1-2018/136005
- WO-A1-2022/080455
- WO-A2-2008/063675
- WO-A2-2010/057039
- WO-A2-2012/087965
- JP-A- 2013 507 932
- JP-A- 2014 501 108
- JP-A- 2015 522 281
- US-A1- 2013 344 594
- WANG YUNFANG ET AL: "Conversion of Human Gastric Epithelial Cells to Multipotent Endodermal Progenitors using Defined Small Molecules", CELL STEM CELL, vol. 19, no. 4, 6 October 2016 (2016-10-06), pages 449 - 461, XP029761238, ISSN: 1934-5909, DOI: 10.1016/J.STEM.2016.06.006
- KE LI ET AL: "Small Molecules Facilitate the Reprogramming of Mouse Fibroblasts into Pancreatic Lineages", CELL STEM CELL, vol. 14, no. 2, 1 February 2014 (2014-02-01), AMSTERDAM, NL, pages 228 - 236, XP055412508, ISSN: 1934-5909, DOI: 10.1016/j.stem.2014.01.006
- TAKESHI KATSUDA ET AL: "Conversion of Terminally Committed Hepatocytes to Culturable Bipotent Progenitor Cells with Regenerative Capacity", CELL STEM CELL, vol. 20, no. 1, 1 January 2017 (2017-01-01), AMSTERDAM, NL, pages 41 - 55, XP055397277, ISSN: 1934-5909, DOI: 10.1016/j.stem.2016.10.007
- MASAKI KAWAMATA AND TAKAHIRO OCHIYA: "Generation of genetically modified rats from embryonic stem cells", PROCEEDINGS OF THE NATIONAL ACADEMY OF SCIENCES, NATIONAL ACADEMY OF SCIENCES, vol. 107, no. 32, 10 August 2010 (2010-08-10), pages 14223 - 14228, XP008153294, ISSN: 0027-8424, [retrieved on 20100726], DOI: 10.1073/PNAS.1009582107
- H. A. RUSS ET AL: "Controlled induction of human pancreatic progenitors produces functional beta-like cells in vitro", THE EMBO JOURNAL / EUROPEAN MOLECULAR BIOLOGY ORGANIZATION, vol. 34, no. 13, 2 July 2015 (2015-07-02), Oxford, pages 1759 - 1772, XP055325317, ISSN: 0261-4189, DOI: 10.15252/embj.201591058
- DAI, PING ET AL.: "Direct Reprogramming by Small Molecules for Regenerative Medicine", JOURNAL OF KYOTO PREFECTURAL UNIVERSITY OF MEDICINE, vol. 127, no. 1, 25 January 2018 (2018-01-25), pages 1 - 12, XP009522958, ISSN: 0023-6012
- KATSUDA, T. ET AL.: "Conversion of Terminally Committed Hepatocytes to Culturable Bipotent Progenitor Cells with Regenerative Capacity", CELL STEM CELL, vol. 20, 2017, pages 41 - 55, XP055397277, DOI: 10.1016/j.stem.2016.10.007
- KATSUDA TAKESHI, OCHIYA TAKAHIRO: "In vitro reprogramming of mature hepatocytes to culturable liver progenitor cells and their potential application to regenerative medicine", REGENERATIVE MEDICINE, vol. 17, no. 1, 28 February 2018 (2018-02-28), JP, pages 78 - 84, XP009527724, ISSN: 1347-7919
- MATSUZAKI, JUNTARO: "Reprogramming of pancreatic exocrine cells into pancreatic progenitor-like cells due to low molecular weight compounds", THE 17TH CONGRESS OF THE JAPANESE SOCIETY FOR REGENERATIVE MEDICINE, vol. 17, 23 March 2018 (2018-03-23), pages 840, XP009529430
- MATSUZAKI, JUNTARO : "O-33-3 Induction of pancreatic progenitor-like cells and insulin- secreting cells of pancreatic exocrine cells due to low molecular weight compounds", THE 18TH CONGRESS OF THE JAPANESE SOCIETY FOR REGENERATIVE MEDICINE, 23 March 2019 (2019-03-23), pages 779, XP009527813

## Description

### TECHNICAL FIELD

The present invention relates to an *in vitro* method for producing pancreatic stem/progenitor cells, and various uses thereof.

### BACKGROUND ART

Although advances in stem cell biology have aroused great interest in its applications in regenerative medicine, they have not yet been realized. Although induced pluripotent stem cells (iPS cells) are one of the most promising cell sources, there still has been no hope of their application to actual clinical practice due to the remaining presence of tumorigenesis risk (Cell. 2014 Oct 9; 159(2):428-39) (Cell Metab. 2016 Apr 12; 23(4): 622-634). Meanwhile, recent study has shown that cells of different lineage can directly be converted (directly reprogrammed) into progenitor cell-like cells, but direct reprogramming is still associated with unexpected risks since it involves genetic modification like iPS cells, and thus cannot be applied to regenerative medicine (Nat Commun. 2016 Jan 6; 7:10080) (Cell Stem Cell. 2016 Mar 3; 18(3):410-21) (Nat Biotechnol. 2014 Dec; 32(12):1223-30).

Recently, there have been reported surprising findings that when the pancreas is injured, proliferative and bipotential pancreatic stem/progenitor cells can be isolated from adult pancreatic exocrine cells (EMBO J. 2013 Oct 16; 32(20):2708-21). These innovative findings provide great insight not only to the pancreatic stem cell theory but also to the pancreatic regeneration studies. Specifically, if such reprogramming can be reproduced *in vitro,* the resulting stem/progenitor cells are expected to serve as a new cell source in regenerative medicine.

However, a method for reprogramming mature cells into stem/progenitor cells without genetic modification is totally unknown.

The inventors of the present invention and other groups have previously reported that a combination of certain types of low molecular weight inhibitors contributes to the induction and maintenance of pluripotency of stem cells (Proc Natl Acad Sci U S A. 2010 Aug 10; 107(32):14223-8) (Cell Stem Cell. 2017 Jan 5; 20(1):41-55) (Cell Stem Cell. 2016 Oct 6; 19(4):449-461).

Moreover, the inventors of the present invention have succeeded in producing hepatic stem/progenitor cells from mature hepatocytes by using a low molecular weight compound (WO2017/119512).

However, except for the case of hepatocytes, there is no report about contribution of these low molecular weight inhibitors to the reprogramming of mature cells into stem/progenitor cells.

### Prior Art Documents

### Patent Documents

Patent Document 1: WO2017/119512

### Non-patent Documents

Non-patent Document 1: Cell. 2014 Oct 9; 159(2):428-39
Non-patent Document 2: Cell Metab. 2016 Apr 12; 23(4): 622-634
Non-patent Document 3: Nat Commun. 2016 Jan 6; 7:10080
Non-patent Document 4: Cell Stem Cell. 2016 Mar 3; 18(3):410-21
Non-patent Document 5: Nat Biotechnol. 2014 Dec; 32(12):1223-30
Non-patent Document 6: EMBO J. 2013 Oct 16; 32(20):2708-21
Non-patent Document 7: Proc Natl Acad Sci U S A. 2010 Aug 10; 107(32): 14223-8
Non-patent Document 8: Cell Stem Cell. 2017 Jan 5; 20(1):41-55
Non-patent Document 9: Cell Stem Cell. 2016 Oct 6; 19(4):449-461

### SUMMARY OF THE INVENTION

### PROBLEM TO BE SOLVED BY THE INVENTION

The objective of the present invention is to provide a method for efficiently reprogramming cells derived from mammalian pancreatic tissue (for example, mature cells) into pancreatic stem/progenitor cells without genetic modification.

### MEANS TO SOLVE THE PROBLEM

In order to achieve the above-described objective, the inventors of the present invention have gone through intensive research, and as a result of which found that when cells derived from mammalian pancreatic tissue are cultured in the presence of a TGFβ-receptor inhibitor, a glycogen synthase kinase 3 (GSK3) inhibitor and a Rho kinase (ROCK) inhibitor, such cells can be reprogrammed into cells that are proliferative and capable of differentiating into pancreatic stem/progenitor cells. The inventors of the present invention have demonstrated that when the pancreatic stem/progenitor cells thus obtained from mature pancreatic exocrine cells are transplanted into diabetic model mice, they can be engrafted as mature pancreatic endocrine cells.

As a result of further studies based on these findings, the inventors of the present invention accomplished the present invention.

Thus, the present invention is as follows.
(1) An *in vitro* method of producing pancreatic stem/progenitor cells, the method comprising contacting cells derived from a mammalian pancreatic tissue with a TGFβ-receptor inhibitor, a GSK3 inhibitor and a ROCK inhibitor.
(2) The *in vitro* method according to (1), wherein the pancreatic tissue comprises pancreatic parenchymal cells.
(3) The *in vitro* method according to (2), wherein pancreatic parenchymal cells comprises pancreatic exocrine cells, and optionally wherein the mammal is a human, rat, or mouse.
(4) The *in vitro* method according to (2) or (3), wherein the contact between the cells, optionally pancreatic exocrine cells, and the TGFβ-receptor inhibitor, and optionally the GSK3 inhibitor and/or the ROCK inhibitor, is carried out by culturing the cells in the presence of the inhibitor(s).
(5) The *in vitro* method according to (2) or (3), wherein the pancreatic tissue comprises pancreatic exocrine cells and the cells are contacted with the TGFβ-receptor inhibitor, the GSK3 inhibitor, and the ROCK inhibitor.
(6) The *in vitro* method according to (5), wherein the TGFβ-receptor inhibitor is A-83-01 (A), the GSK3 inhibitor is CHIR99021 (C), and the ROCK inhibitor is Y-27632 (Y).
(7) A method for maintaining or expanding pancreatic stem/progenitor cells comprising:
   (i) producing pancreatic stem/progenitor cells by the *in vitro* method of any of (1) to (6); and
   (ii) subculturing the pancreatic stem/progenitor cells on a collagen- or Matrigel-coated culture vessel in the presence of the TGFβ-receptor inhibitor, a GSK3 inhibitor and a ROCK inhibitor.
(8) A method for inducing pancreatic stem/progenitor cells into cells derived from a mammalian endodermal tissue or organ or into pancreatic exocrine cells, comprising:
   (i) producing pancreatic stem/progenitor cells by the *in vitro* method of any of (1) to (6); and
   (ii) culturing the pancreatic stem/progenitor cells in the presence of a TGFβ-receptor inhibitor, a GSK3 inhibitor and a ROCK inhibitor.
(9) A *in vitro* method for assessing the metabolism of a test compound, comprising the steps of:
   (i) producing pancreatic stem/progenitor cells by the *in vitro* method of any of (1) to (6);
   (ii) contacting the test compound with the pancreatic stem/progenitor cells; and
   (iii) measuring the metabolism of the test compound by the cells.
(10) An *in vitro* method for screening for secretion inducers of enzymes secreted from cells comprising the steps of:
   (i) producing pancreatic stem/progenitor cells by the *in vitro* method of any of (1) to (6);
   (ii) contacting a test compound with the pancreatic stem/progenitor cells; and
   (iii) measuring the substance(s) secreted by the cells.
(11) An *in vitro* method for assessing the endodermal tissue or organ toxicity of a test compound, comprising the steps of:
   (i) producing pancreatic stem/progenitor cells by the *in vitro* method of any of (1) to (6);
   (ii) contacting the test compound with the pancreatic stem/progenitor cells; and
   (iii) measuring the presence or the absence, or the degree of damage to the cells contacted with the test compound.
(12) Use of an inducer comprising a TGFβ-receptor inhibitor in an *in vitro* method of inducing cells derived from mammalian pancreatic tissue into pancreatic stem/progenitor cells, wherein the inducer further comprises a GSK3 inhibitor and a ROCK inhibitor.
(13) Use according to (12), wherein the pancreatic tissue comprises pancreatic parenchymal cells optionally wherein the pancreatic parenchymal cells comprise pancreatic exocrine cells, and optionally wherein the mammal is a human, a rat, or a mouse.

### EFFECTS OF THE INVENTION

According to the present invention, pancreatic stem/progenitor cells having self-renewal ability and differentiation potency (bipotency) into pancreatic endocrine cells can safely and rapidly be induced from mature cells (e.g., pancreatic exocrine cells) without genetic modification.

### BRIEF DESCRIPTION OF THE DRAWINGS

Figure 1 shows changes in cell morphology upon induction of pancreatic progenitor cells.
Figure 2 shows changes in mRNA expression of pancreatic progenitor cell markers (mouse) upon induction of pancreatic progenitor cells.
Figure 3 shows mRNA expression of pancreatic progenitor cell markers (rat) after the first passage of subculture.
Figure 4 shows clusters of insulin-secreting cells.
Figure 5 shows mRNA expression of β cell marker molecules and C-peptide secretion in clusters of insulin-secreting cells.
Figure 6 shows mRNA expression of Insulin and GLUT2.
Figure 7 shows the responsiveness of pancreatic endocrine cells to glucose concentrations.
Figure 8 shows mRNA expression of pancreatic progenitor cell markers after the first passage of subculture.
Figure 9 shows histological images of transplanted cells in an *in vivo* diabetic environment.

### DESCRIPTION OF EMBODIMENTS

### 1. Summary

The present invention provides an *in vitro* method of producing pancreatic stem/progenitor cells, the method comprising contacting cells derived from mammalian pancreatic tissue with a TGFβ-receptor inhibitor, a GSK3 inhibitor and a ROCK inhibitor.

As described above, a method for producing hepatic stem/progenitor cells from mature hepatocytes by using a low molecular weight compound has been successfully provided by the inventors of the present invention (WO2017/119512). However, it is uncertain whether not only hepatocytes, but also other mature cells from endodermal organs or tissues can be reprogrammed into stem cells or progenitor cells (hereinafter referred to as "stem/progenitor cells").

When using cells from the pancreas as non-hepatocyte cells, the inventors of the present invention have succeeded in their reprogramming into pancreatic stem cells or pancreatic progenitor cells (hereinafter referred to as "pancreatic stem/progenitor cells") by the action of a low molecular weight compound.

As used herein, the term "stem cells" refers to cells that have self-renewal ability and pluripotency for differentiation into various cells, while the term "progenitor cells" refers to cells that develop from stem cells and are at an intermediate stage of differentiation into particular types of terminally differentiated cells constituting the body. These stem cells or progenitor cells are collectively referred herein to as "stem/progenitor cells" and are expressed as "pancreatic stem/progenitor cells" if the cells to be reprogrammed are pancreatic cells.

In the present invention, the "endoderm" is one of the three germ layers that arise during the development of metazoans, and embryologically constitutes the whole or part of the primitive gut wall during primitive gut formation (at the gastrula stage). The endoderm develops into the main part of the digestive tract and its accessory glands (liver, pancreas), thyroid gland, lung and other respiratory organs, etc.

In the present invention, the "endodermal tissue or organ" is exemplified by the digestive tract (esophagus, stomach, small intestine, large bowel), lung, pancreas, thyroid gland, parathyroid gland, larynx, trachea, bronchus, urinary bladder, urethra, prostate and so on (provided that the liver is excluded).

On the other hand, there are over 2,000 types of enzymes in the liver, and metabolic reactions are continuously carried out in the liver to chemically remake various substances. This is the reason why the liver is called "chemical factory in the body" and the liver greatly differs from any other endodermal tissues such as pancreatic beta cells having only the function of producing a single hormonal substance called insulin and a series of digestive system cells belonging to the endoderm. The liver shows the highest regeneration ability in the body, and when the liver and other endodermal tissues are compared for the degree of difficulty in their reprogramming, the endodermal tissues other than the liver are more significantly difficult to reprogram. Namely, once endoderm-derived cells have differentiated into mature cells, they will have characteristics peculiar to each organ in terms of proliferation ability, metabolic ability, humoral factor secretion ability, etc.; and hence it has been previously unknown whether the events found in mature hepatocytes are specific to mature hepatocytes or ubiquitous in endoderm-derived mature cells.

The inventors of the present invention have found that reprogramming from mature cells into stem/progenitor cells is also possible for endodermal tissues or organs other than the liver.

The present invention is characterized by bringing cells derived from a mammalian pancreatic tissue into contact *in vitro* with a TGFβ-receptor inhibitor, a glycogen synthase kinase 3 (GSK3) inhibitor and a Rho kinase (ROCK) inhibitor. This allows, starting from mature cells derived from pancreatic tissue, to produce pancreatic stem/progenitor cells. The method of the present invention is also referred to as "the reprogramming method of the present invention."

### 2. Induction of pancreatic stem/progenitor cells from mature cells

The present invention provides an *in vitro* method for producing pancreatic stem/progenitor cells, the method comprising contacting cells derived from mammalian pancreatic tissue with a TGFβ-receptor inhibitor, a glycogen synthase kinase 3 (GSK3) inhibitor and a Rho kinase (ROCK) inhibitor.

To produce or induce pancreatic stem/progenitor cells from mature pancreatic exocrine cells, the present invention comprises bringing mammalian pancreatic exocrine cells into contact *in vitro* with low molecular weight signaling pathway inhibitors including a TGFβ-receptor inhibitor, a glycogen synthase kinase 3 (GSK3) inhibitor and a Rho kinase (ROCK) inhibitor.

The cells derived from mammalian endodermal pancreatic tissue (hereinafter also referred to as "mature cells") used as a starting material for the reprogramming method of the present invention are exemplified by digestive tract epithelial cells, alveolar epithelial cells, pancreatic parenchymal cells, thyroid gland follicular epithelial cells, urinary tract epithelial cells, prostate epithelial cells, etc. In the case of using pancreatic parenchymal cells as a starting material, pancreatic exocrine cells, pancreatic endocrine cells or the like can be used. "Pancreatic exocrine cells" are classified into acinar cells and pancreatic duct cells, either or both of which are deemed to serve as a starting source for pancreatic stem/progenitor cells. Likewise, "pancreatic endocrine cells" are classified into α cells, β cells, δ cells, ε cells and PP cells, any or all of which are deemed to serve as a starting source for pancreatic stem/progenitor cells.

The cells used for the reprogramming method of the present invention may be provided from any mammalian pancreatic tissue and examples include mammalian (e.g., human, rat, mouse, guinea pig, rabbit, sheep, horse, pig, bovine, monkey or the like, preferably human, rat or mouse) embryonic stem cells (ES cells) or pluripotent stem cells such as iPS cells.

However, considering that the main problem of the present invention is to safely and rapidly provide pancreatic stem/progenitor cells without genetic modification, for example in the case of using pancreatic exocrine cells, those isolated/purified from a pancreas removed from a mammal are used.

For example, in a case of a rat, a pancreas removed from a 10- to 20-week-old adult rat is preferably used, although a pancreas derived from a juvenile rat less than 2-month-old may also be used. In a case of a human, a pancreas may be obtained by biopsy or surgical operation. For example, pancreatic parenchyma, digestive tract epithelium, alveolar epithelium, prostatic epithelium and others may all be taken by biopsy. In the case of surgical operation, it is possible to use a pancreatic tissue piece sectioned from an adult or a pancreas sectioned from an aborted fetus. Alternatively, cells obtained by cryopreserving these isolated/purified pancreatic exocrine cells removed from the pancreas (cryopreserved pancreatic exocrine cells) may also be used.

For purification of cells derived from a mammalian endodermal tissue or organ, the tissue is isolated and enzymatically treated, followed by filtration, centrifugation, etc., to purify the cells.

For purification of pancreatic exocrine cells from a mammalian pancreas or a tissue piece thereof, the pancreatic tissue is isolated and digested with collagenase, followed by filtration, centrifugation, etc., to remove Langerhans' islets, non-parenchymal cells and cell debris, thereby purifying pancreatic exocrine cells.

The cells derived from mammalian pancreatic tissue (e.g., pancreatic exocrine cells) prepared as described above are brought into contact *in vitro* with low molecular weight signaling pathway inhibitors including a TGFβ-receptor inhibitor, a GSK3 inhibitor and a ROCK inhibitor

The TGFβ-receptor inhibitor used for the present invention may be any inhibitor as long as it inhibits the function of the transforming growth factor (TGF) β-receptor, where examples include 2-(5-benzo[1,3]dioxole-4-yl-2-tert-butyl-1H-imidazol-4-yl)-6-methylpyridine, 3-(6-methylpyridine-2-yl)-4-(4-quinolyl)-1-phenylthiocarbamoyl-1H-pyrazole (A-83-01), 2-(5-chloro-2-fluorophenyl)pteridine-4-yl)pyridine-4-ylamine (SD-208), 3-(pyridine-2-yl)-4-(4-quinonyl)]-1H-pyrazole, 2-(3-(6-methylpyridine-2-yl)-1H-pyrazole-4-yl)-1,5-naphthyridine (all from Merck) and SB431542 (Sigma Aldrich). A preferable example includes A-83-01. The TGFβ-receptor inhibitor also comprises a TGFβ-receptor antagonist.

The TGFβ-receptor inhibitor may be one type of compound or a combination of two or more types compounds.

Examples of a low molecular weight signaling pathway inhibitor other than the TGFβ-receptor inhibitor include a GSK3 inhibitor and a ROCK inhibitor.

The GSK3 inhibitor used for the present invention may be any inhibitor as long as it inhibits the function of glycogen synthase kinase (GSK) 3, where examples include SB216763 (Selleck), CHIR98014, CHIR99021 (all from Axon medchem), SB415286 (Tocris Bioscience), and Kenpaullone (Cosmo Bio). A preferable example includes CHTR99021.

The GSK3 inhibitor may be one type of compound or a combination of two or more types compounds.

The ROCK inhibitor used for the present invention may be any inhibitor as long as it inhibits the function of Rho-binding kinase. Examples of the ROCK inhibitor include GSK269962A (Axon medchem), Fasudil hydrochloride (Tocris Bioscience), Y-27632 and H-1152 (all from Wako Pure Chemical). A preferable example includes Y-27632.

The ROCK inhibitor may be one type of compound or a combination of two or more types compounds.

When the GSK3 inhibitor and the ROCK inhibitor are used alone or in combination and brought together with the TGFβ-receptor inhibitor into contact with the cells derived from mammalian pancreatic tissue (e.g., pancreatic exocrine cells), the efficiency of inducing pancreatic stem/progenitor cells (also referred to as "reprogramming efficiency") is significantly increased as compared to the case where only the TGFβ-receptor inhibitor is brought into contact with the cells. Therefore, according to the reprogramming method of the present invention, the GSK3 inhibitor and the ROCK inhibitor, in addition to the TGFβ-receptor inhibitor, is brought into contact with the cells derived from mammalian pancreatic tissue (e.g., pancreatic exocrine cells).

In the present invention, combinations of the TGFβ-receptor inhibitor and the GSK3 inhibitor and the ROCK inhibitor are shown below.
(a) A-83-01 (A) as the TGFβ-receptor inhibitor in combination with CHIR99021 (C) as the GSK3 inhibitor (AC) and a ROCK inhibitor.
(b) A-83-01 (A) as the TGFβ-receptor inhibitor in combination with Y-27632 (Y) as the ROCK inhibitor (YA) and a GSK3 inhibitor.
(c) A-83-01 (A) as the TGFβ-receptor inhibitor in combination with CHIR99021 (C) as the GSK3 inhibitor and Y-27632 (Y) as the ROCK inhibitor (YAC).

When the GSK3 inhibitor and the ROCK inhibitor are used in combination with the TGFβ-receptor inhibitor, the difference in the reprogramming effect is small from that obtained with a combination of the TGFβ-receptor inhibitor and the GSK3 inhibitor, but the former gives better proliferation ability of the resulting pancreatic stem/progenitor cells than the latter. Therefore, in accordance with the present invention, a combination of the TGFβ-receptor inhibitor, the GSK3 inhibitor and the ROCK inhibitor is brought into contact with the mature cells (e.g., pancreatic exocrine cells).

According to the reprogramming method of the present invention, contact between cells derived from mammalian pancreatic tissue (which may be mature cells such as pancreatic exocrine cells) and the low molecular weight signaling pathway inhibitors including the TGFβ-receptor inhibitor, GSK3 inhibitor and the ROCK inhibitor can be carried out by culturing the cells in the presence of these inhibitors. Specifically, these inhibitors are added to a medium at an effective concentration to carry out the culturing. As this medium, a medium widely used for culturing animal cells may be utilized as a basal medium. A commercially available basal medium may also be employed, where examples include, but not particularly limited to, a minimum essential medium (MEM), a Dulbecco's modified minimum essential medium (DMEM), a RPMI1640 medium, a 199 medium, a Ham's F12 medium and a William's E medium, which may be used alone or two or more types of them may be used in combination.

Examples of additives to the medium include various amino acids (for example, L-glutamine, L-proline, etc.), various inorganic salts (salt of selenious acid, NaHCO₃, etc.), various vitamins (nicotinamide, ascorbic acid derivative, etc.), various antibiotics (for example, penicillin, streptomycin, etc.), an antimycotic agent (for example, amphotericin, etc.), and buffers (HEPES, etc.).

In addition, a 5-20% serum (FBS, etc.) may be added to the medium, or the medium may be a serum-free medium. In a case of a serum-free medium, a serum substitute (BSA, HAS, KSR, etc.) may be added. In general, a factor such as a growth factor, cytokine or hormone is further added. Examples of such factors include, but not limited to, epidermal growth factor (EGF), insulin, transferrin, hydrocortisone 21-hemisuccinate or a salt thereof and dexamethasone (Dex).

The concentration of the TGFβ-receptor inhibitor added to the medium may suitably be selected, for example, in a range of 0.01-10 µM, and preferably 0.1-1 µM.

The concentration of the GSK3 inhibitor added to the medium may suitably be selected, for example, in a range of 0.01-100 µM, and preferably 1-10 µM.

The concentration of the ROCK inhibitor added to the medium may suitably be selected, for example, in a range of 0.0001-500 µM, and preferably 1-50 µM.

When these inhibitors are water-insoluble or poorly water-soluble compounds, they may be dissolved in a small amount of a low-toxicity organic solvent (for example, DMSO, etc.), and then the resultant can be added to a medium to give the above-described final concentration.

The culture vessel used for this culture is not particularly limited as long as it is suitable for adhesion culture, where examples include a dish, a petri dish, a tissue culture dish, a multidish, a microplate, a microwell plate, a multiplate, a multiwell plate, a chamber slide, a Schale, a tube, a tray, and a culture bag. For floating cell culture, the culture vessel used may have its surface treated to avoid cell adhesion. Alternatively, in the case of adhesion culture, the culture vessel used may have its inner surface coated with a cell supporting substrate for the purpose of enhancing adhesiveness with the cells. Examples of such a cell supporting substrate include collagen, gelatin, Matrigel, poly-L-lysine, laminin and fibronectin, and preferably collagen and Matrigel.

The mature cells can be seeded onto a culture vessel at a cell density of 10²-10⁶ cells/cm², and preferably 10³-10⁵ cells/cm².

In the case of pancreatic exocrine cells, they can also be seeded onto a culture vessel at a cell density of 10²-10⁶ cells/cm², and preferably 10³-10⁵ cells/cm². Culture can take place in a CO₂ incubator, in an atmosphere at a CO₂ concentration of 1-10%, preferably 2-5% and more preferably about 5%, at 30-40°C, preferably 35-37.5°C and more preferably about 37°C. The culture period may be, for example, 1-4 weeks, and preferably 1-3 weeks. The medium is freshly exchanged every 1-3 days.

In this manner, the mature cells (e.g. pancreatic exocrine cells) are brought into contact with the TGFβ-receptor inhibitor, and the GSK3 inhibitor and the ROCK inhibitor so as to reprogram the mature cells into pancreatic stem/progenitor cells. For example, once primary mouse mature pancreatic exocrine cells have been cultured with A-83-01 as the TGFβ-receptor inhibitor (A) in combination (YAC) with CHIR99021 as the GSK3 inhibitor (C) and Y-27632 as the ROCK inhibitor (Y), they will proliferate by about 3000 times by 30 days of culture and show a significant increase as compared to culture in the absence of YAC.

As used herein, the term "pancreatic stem/progenitor cells" (hereinafter also referred to as "PSCs") refers to stem cells or progenitor cells which have (a) self-regeneration ability and (b) the ability to differentiate into pancreatic endocrine cells (e.g., cells constituting Langerhans' islets) or pancreatic exocrine cells.

The pancreatic stem/progenitor cells (PSCs) also include pancreatoblasts from a fetal pancreas.

According to one preferable embodiment, in addition to the features (a) and (b) above, PSCs obtained by the reprogramming method of the present invention (c) express master factors Pdx1 and Nkx6.1, and also express Gata4, Hes1, Sox9, Foxa2, CK19, CD133 and so on. However, PSCs obtained by the reprogramming method of the present invention do not express Lgr5 which is expressed in other known PSCs.

PSCs prepared by methods of the present invention further have one or more of the following features.

(d) the apparent growth rate does not slow down for at least 10 passages, preferably 20 passages or more of subculture.

(e) differentiation potency into pancreatic endocrine cells is retained for at least 10 passages, preferably 20 passages or more of subculture.

(f) nuclear cytoplasmic (N/C) ratio is higher than that of pancreatic exocrine cells.

(g) expressions of one or more PSC marker genes selected from Pdx1 and Nkx6.1 are increased compared to pancreatic exocrine cells.

(h) expressions of one or more proteins selected from Pdx1 and Nkx6.1 are increased compared to pancreatic exocrine cells.

According to a preferable embodiment, PSCs prepared by a method of the present invention have all of the above-described features (d)-(h).

As described above, when brought into contact with a TGFβ-receptor inhibitor, a GSK3 inhibitor and a ROCK inhibitor, mature cells derived from mammalian pancreatic tissue (e.g., pancreatic exocrine cells) can be induced into pancreatic stem/progenitor cells (e.g., pancreatic exocrine cells can be induced into PSCs).

Therefore, the present invention provides a method for inducing pancreatic stem/progenitor cells into cells derived from a mammalian endodermal tissue or organ or into pancreatic exocrine cells , comprising: (i) producing pancreatic stem/progenitor cells by the *in vitro* method of the present invention; and (ii) culturing the pancreatic stem/progenitor cells in the presence of a TGFβ-receptor inhibitor, a GSK3 inhibitor and a ROCK inhibitor.

The present invention provides a method for inducing pancreatic exocrine cells into PSCs by bringing the pancreatic exocrine cells into contact with a TGFβ-receptor inhibitor, a GSK3 inhibitor and a ROCK inhibitor. The present invention also provides for the use of a PSC inducer comprising a TGFβ-receptor inhibitor for inducing cells derived from mammalian pancreatic tissue (such as pancreatic exocrine cells) into PSCs, wherein the inducer comprises a combination of a TGFβ-receptor inhibitor, a GSK3 inhibitor and a ROCK inhibitor.

While the TGFβ-receptor inhibitor, the GSK3 inhibitor and the ROCK inhibitor can directly be used as a PSC inducer, they may also be made into a liquid agent by dissolving them in a suitable solvent. Alternatively, these inhibitors can be made into a kit by combining with the above-described medium for inducing PSCs from pancreatic exocrine cells.

### 3. Maintenance and expansion of pancreatic stem/progenitor cells

The pancreatic stem/progenitor cells produced according to methods of the present invention, as described above, can be efficiently maintained or expanded by being subcultured on a collagen- or Matrigel-coated culture vessel in the presence of a TGFβ-receptor inhibitor, a GSK3 inhibitor and a ROCK inhibitor.

As the culture vessel, a culture vessel similar to one used for inducing mature cells into pancreatic stem/progenitor cells can be used.

In the case of using pancreatic exocrine cells, PSCs obtained by the method of the present invention can be efficiently maintained or expanded by being subcultured on a collagen- or Matrigel-coated culture vessel in the presence of a TGFβ-receptor inhibitor, a GSK3 inhibitor and a ROCK inhibitor.

As the culture vessel, a culture vessel similar to one used for inducing pancreatic exocrine cells into PSCs can be used.

Once the primary PSCs obtained as described above have reach 70-100% confluency, they are seeded onto this collagen- or Matrigel-coated culture vessel at a density of 10³-10⁵ cells/cm². As the medium, the medium described for induction culture of PSCs can similarly be used. The concentrations of the TGFβ-receptor inhibitor, the GSK3 inhibitor and the ROCK inhibitor added can also suitably be selected from the concentration ranges described above for induction culture of PSCs. The culture temperature and the CO₂ concentration also follow the conditions for induction culture of PSCs. Once 70-100% confluency has been reached, the cells are treated with trypsin to be dissociated, and subcultured. Stable PSCs can be obtained after about 5-8 passages of subculture. After 10 passages or more of subculture, cloning can be conducted by a routine procedure.

As described above, the TGFβ-receptor inhibitor, the GSK3 inhibitor and the ROCK inhibitor are added to the medium not only for induction culture but also for maintenance or expansion culture of pancreatic stem/progenitor cells (PSCs). Thus, an agent for maintaining or expanding stem/progenitor cells (e.g., PSCs) comprises a TGFβ-receptor inhibitor, a GSK3 inhibitor and a ROCK inhibitor.

### 4. Differentiation from pancreatic stem/progenitor cells into mature cells

Induction of differentiation from pancreatic stem/progenitor cells into mature cells may be accomplished, for example, by floating culture in SHM (Small hepatocyte medium) supplemented with YAC on an ultra-low adsorption culture dish for 7 days. Alternatively, it is also possible to use, e.g., a method for culture in a culture solution supplemented with Phorbol 12,13-dibutyrate, LDN193189, Keratinocyte Growth Factor, SANT1, Retinoic Acid, XXI, Betacellulin and so on (Cell. 2014 Oct 9; 159(2):428-39).

Induction of differentiation from PSCs into pancreatic endocrine cells may also be accomplished in the same manner as described above. Alternatively, it is also possible to use, e.g., a method for culture in a culture solution supplemented with Phorbol 12,13-dibutyrate, LDN193189, Keratinocyte Growth Factor, SANT1, Retinoic Acid, XXI, Betacellulin and so on (Cell. 2014 Oct 9; 159(2):428-39).

When PSCs produced by the methods of the present invention are induced to differentiate, the resulting pancreatic exocrine cells have the insulin-producing function typical of mature pancreatic endocrine cells, and C-peptide secretion reflecting insulin secretion is also detected. Moreover, increases in mRNA levels are observed for Ngn3 and glucose transporter (GLUT2) which are master transcription factors in pancreatic endocrine cells. Namely, PSCs produced by the methods of the present invention are capable of differentiating into functional pancreatic endocrine cells.

### 5. Application of the pancreatic stem/progenitor cells produced according to methods of the present invention

The mature cells redifferentiated from the pancreatic stem/progenitor cells produce of the present invention as described in Item 4 above can be utilized, for example, for assessing metabolism and cell or tissue toxicity of a test compound.

Conventionally, animal models or the like have been used for the assessment of metabolism and toxicity of a test compound, but there are problems like limitation in the number of the test compounds that can be assessed at one time and assessments obtained with animal models or the like being unable to directly be applied to human. Therefore, an assessment method using a human cancer cell line or a primary culture of normal human cells has been employed. However, assessment obtained with the human cancer cell line may possibly be inapplicable to normal human cells. In addition, the primary cultures of normal human cells are associated with problems in terms of stable supply and cost. Moreover, cell lines obtained by immortalizing primary cultures of normal human cells are shown to have lower activity as compared to those not immortalized. These problems may be solved by utilizing cells produced according to the method of the present invention.

Thus, the present invention provides an *in vitro* method for assessing the metabolism of a test compound, comprising the steps of: producing pancreatic stem/progenitor cells by the *in vitro* method of the present invention; contacting a test compound with the pancreatic stem/progenitor cells; and measuring the metabolism of the test compound in the cells.

Thus, in the case of using pancreatic exocrine cells in the method of the present invention, a test compound is brought into contact with pancreatic exocrine cells produced by the *in vitro* method of the present invention, followed by measuring the metabolism of the test compound by the cells.

The test compound used with the present invention is not particularly limited. Examples include, but not limited to, a xenobiotic substance, a natural compound, an organic compound, an inorganic compound, a protein, a single compound such as a peptide, an expression product from a compound library or a gene library, a cell extract, a cell culture supernatant, a fermentative microbial product, a marine organism extract and a plant extract.

Examples of the xenobiotic substance include, but not limited to, candidate compounds for drugs and food, existing drugs and food, and the xenobiotic substance intended in the present invention comprises any substance as long as it is a foreign matter to the living body. More specific examples include Rifampin, Dexamethasone, Phenobarbital, Ciglirazone, Phenytoin, Efavirenz, Simvastatin, β-Naphthoflavone, Omeprazole, Clotrimazole and 3-Methylcholanthrene.

Contact between mature cells (e.g., pancreatic exocrine cells) and a test compound is usually carried out by adding the test compound to a medium or a culture solution, but it is not limited thereto. If the test compound is a protein or the like, a DNA vector expressing said protein may be introduced into the cells to make contact therewith.

The metabolism of the test compound can be measured by a method well known to those skilled in the art. For example, the test compound is judged to have been metabolized if a metabolite of the test compound is detected.

For example, the test compound is also judged to have been metabolized if expression of an enzyme gene such as insulin is induced or activity of such enzyme is increased upon contact with the test compound.

The present invention also provides an *in vitro* screening method for secretion inducers of enzymes secreted from cellscomprising the steps of: producing pancreatic stem/progenitor cells by the *in vitro* method of the present invention; contacting a test compound with the pancreatic stem/progenitor cells; and measuring the substance(s) secreted in the cells.

For example, screening for secretion modulators can be made on the basis of whether upon contact with a test substance, pancreatic stem/progenitor cells or induced pancreatic cells are induced to secret each enzyme to be secreted therefrom.

The present invention also provides an *in vitro* method for assessing the endodermal tissue or organ toxicity of a test compound, comprising the steps of: producing pancreatic stem/progenitor cells by the *in vitro* method of the present invention; contacting the test compound with the pancreatic stem/progenitor cells; and measuring the presence or the absence, or the degree of damage to the cells contacted with the test compound.

In the case of assessing the pancreatic toxicity of a test compound in the present invention, the test compound is brought into contact with pancreatic exocrine cells produced by the *in vitro* method of the present invention, followed by measuring the degree of damage in the pancreatic exocrine cells contacted with the test compound. The degree of damage may be measured, for example, on the basis of the viability of pancreatic exocrine cells or pancreatic damage markers.

For example, a test compound is judged to have pancreatic toxicity if the viability of pancreatic exocrine cells is decreased upon adding the test compound to the culture solution of the pancreatic exocrine cells, whereas a test compound is judged to have no pancreatic toxicity when there is no significant change in the viability.

Here, a compound whose presence or absence of pancreatic toxicity is already known can be used as a control so as to assess whether or not a test compound has pancreatic toxicity in a more accurate way.

Further, disclosed herein but not forming part of the claimed invention, is an agent for ameliorating endodermal tissue or organ damage, which comprises the stem/progenitor cells obtained by the *in vitro* method disclosed herein, the pancreatic stem/progenitor cells obtained by the *in vitro* method disclosed herein or the cells induced by the induction method disclosed herein. Furthermore, disclosed herein but not forming part of the claimed invention, the above method, comprises administering a mammal having endodermal tissue or organ damage with an effective amount of the pancreatic stem/progenitor cells obtained by the method of the present invention, or the cells induced by the induction method of the present invention.

For example, to ameliorate pancreatic damage, PSCs produced by a method of the present invention can be transplanted into an immunodeficient mouse with chronic pancreatic damage so as to exert pancreatic regeneration ability comparative to transplantation of primary mature pancreatic exocrine cells. Thus, disclosed herein but not forming part of the claimed invention, is an agent for ameliorating pancreatic damage, which comprises PSCs produced by a method of the present invention.

*If necessary, PSCs produced by methods of the present invention may be purified before* use by flow cytometry using an antibody against a surface antigen marker. PSCs can be suspended in a suitable isotonic buffer (for example, PBS) to be formulated. If necessary, a pharmaceutically acceptable additive can further be contained. Although the PSC suspension may differ depending on the type of pancreatic disease, the severity of pancreatic damage or the like, for example, 10⁸-10¹¹ cells can be transplanted in a case of an adult.

Hereinafter, the present invention will be described more specifically by way of examples, although the present invention should not be limited to these examples in any way.

### EXAMPLES

### 1. Experimental procedures

### Establishment of pancreatic progenitor cells

Mouse or rat pancreatic exocrine cells were treated with collagenase and isolated in accordance with known techniques (Reichert et al. Cold Spring Harb Protoc. 2015 Jun 1; 2015(6):558-61). Dithizone (DTZ) staining was conducted to confirm the absence of insulin-secreting cells among the isolated cells. The isolated pancreatic exocrine cells were plated on a type I collagen-coated culture dish using Small hepatocyte medium (SHM) supplemented with three types of low molecular weight compounds (Y-27632 [10 µM], A-83-01 [0.5 µM], CHIR99021 [3 µM]; hereinafter referred to as YAC).

SHM medium: DMEM/F12 (472.7 mL) supplemented with the additives shown below (Katsuda et al. Bio Protoc. 2018 Jan 20; 8(2)):
2 M HEPES, 1.25 mL
30 g/L L-proline, 500 µL
100x antibiotic/antimycotic, 5 ml
5 N NaOH, 250 µL (for adjustment to pH 7.5)
5% BSA, 5 mL
10 µg/mL EGF, 500 µL
100x ITS-X, 5 mL
10⁻⁴ M dexamethasone, 500 µL
1 M nicotinamide, 5 mL
100 mM Asc2P, 5 mL

### Subculture of PSCs

On the 14th day of primary culture, the cells cultured in the presence of YAC were collected by treatment with trypsin, and then seeded at 9 × 10³ cells/cm² in SHM supplemented with YAC. The mouse cells were cultured on a type I collagen-coated culture dish, while the rat cells were cultured on a Matrigel-coated culture dish. CELLBANKER^{®} 1 (TaKaRa Shuzo, Otsu) was used to prepare cryopreserved stocks. After at least 10 passages of subculture, PSCs were cloned using a BD FACSAria II cell sorter.

### Low-speed imaging at low cell density

The primary pancreatic exocrine cells were seeded onto a collagen-coated 35-mm plate (IWAKI) in the presence or the absence of YAC at 1 × 10² cells/cm². On the first day, the medium was exchanged. After the second medium exchange, BZ9000 All-in-One Fluorescence Microscope (Keyence, Osaka) was used to perform low-speed imaging. Phase difference images were taken every 30 minutes for 300 times from Day 2 to Day 6, and movies were made for every analytical field. Next, individual cells were traced throughout the imaging period to determine the final cell count originating from the cells of interest. Additionally, the total number of apoptotic cells originating from the individual cells was also counted to quantitate apoptotic frequency as total apoptotic cells/original total cell count (counted at the beginning of low-speed imaging).

### Quantitative RT-PCR

Total RNA was isolated from the pancreatic exocrine cells and PSC cells using miRNeasy Mini Kit (QIAGEN). Reverse transcription reaction was carried out using High-Capacity cDNA Reverse Transcription Kit (Life Technologies) following the manufacturer's guideline. The resulting cDNA was used as a template to perform PCR with Platinum SYBR Green qPCR SuperMix UDG (Invitrogen). The expression level of the target gene was normalized with β-actin as the endogenous control.

### Immunocytochemistry and immunohistochemistry

The cells were fixed with 4% paraformaldehyde for 15 minutes. The resultant was incubated with a blocking solution (Blocking One) (Nacalai Tesque, Kyoto) at 4°C for 30 minutes, and then the cells were incubated with primary antibody at room temperature for an hour or at 4°C overnight. Then, Alexa Fluor 488- or Alexa Fluor 594-labeled secondary antibody (Life Technologies) was used to detect the primary antibody. The nuclei were co-stained with Hoechst 33342 (Dojindo).

The tissue sample was fixed with formalin and paraffin-embedded. After dewaxing and rehydration, the specimen was boiled in a 1/200 diluted ImmunoSaver (Nisshin EM, Tokyo) at 98°C for 45 minutes to retrieve the heat-induced epitope. Then, the specimen was treated with 0.1% Triton-X 100 for membrane permeabilization. Following treatment with a blocking reagent (Nacalai Tesque) at 4°C for 30 minutes, the specimen was incubated with a primary antibody at room temperature for an hour. These sections were stained using ImmPRESS IgG-peroxidase kit (Vector Labs) and metal-enhanced DAB substrate kit (Life Technologies) following the manufacturers' instructions. After counterstaining with hematoxylin, the specimen was dehydrated and mounted.

### Induction from PSCs into pancreatic endocrine cells

The cells were suspended in 5% FBS-containing SHM + YAC (cell density: 1 × 10⁴ to 1 × 10⁶ cells/cm²) and cultured on an ultra-low adsorption culture dish for 7 days. The medium was exchanged on the fourth day.

### 2. Results

### 1) Establishment of pancreatic progenitor cells

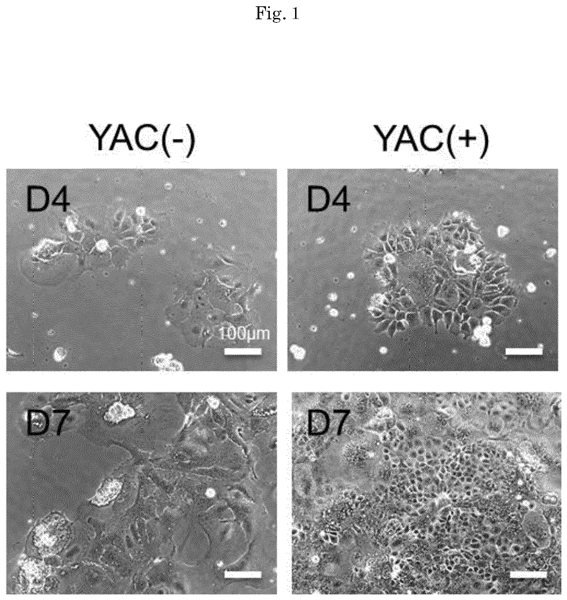

A medium containing pancreatic exocrine cells was supplemented with YAC to thereby establish small epithelial cells having high proliferation ability as shown in Figure 1. These cells were able to be subcultured for over 20 passages and were able to be grown in single cell culture. Moreover, these cells are capable of differentiating into endocrine system cells having the ability to synthesize insulin, and are therefore regarded as having characteristics as pancreatic progenitor cells.

### 2) Properties of induced pancreatic progenitor cells

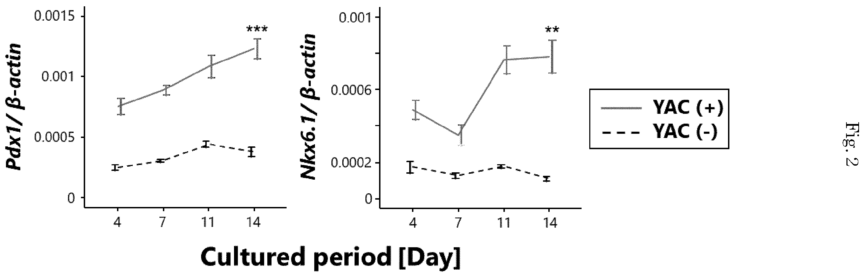

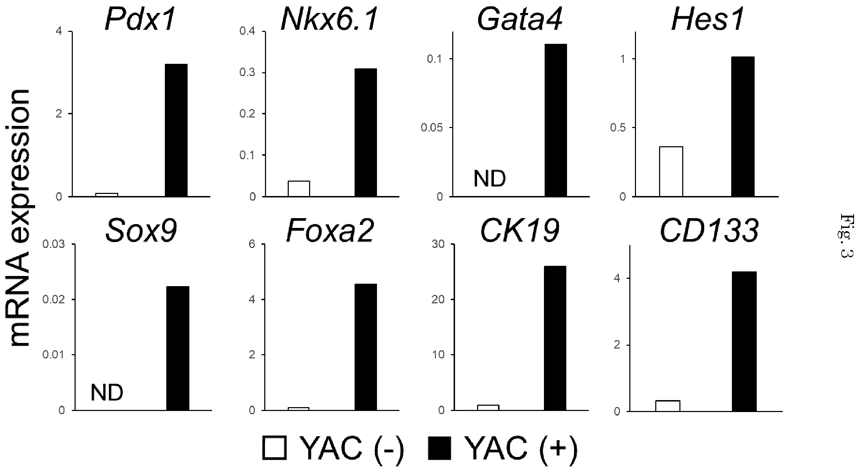

The establish cells were found to express stem cell markers including Pdx1 and Nkx6.1 which are master transcription factors in pancreatic progenitor cells, as shown in Figures 2 and 3.

### 3) Induction of differentiation from pancreatic progenitor cells into insulin-producing cells

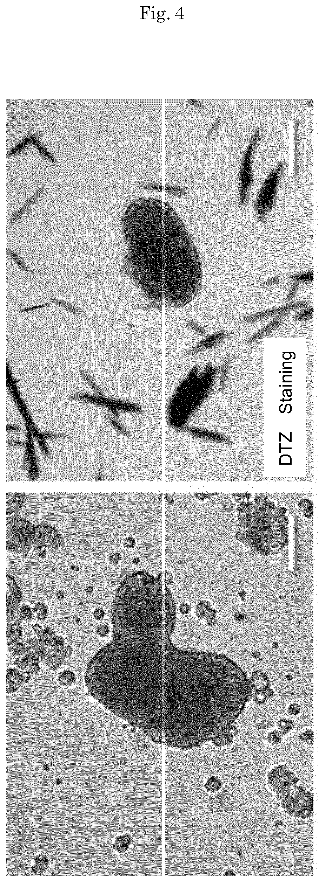

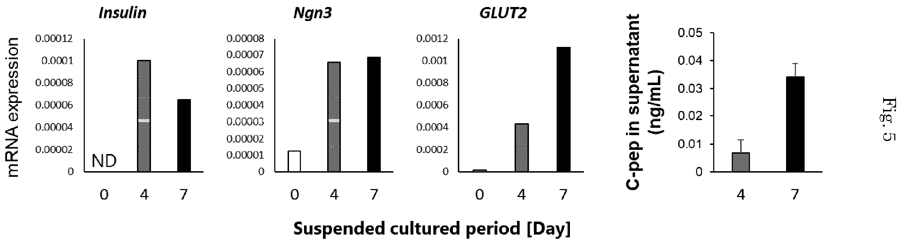

Upon floating culture for 7 days using SHM medium supplemented with YAC on an ultra-low adsorption culture dish, the established pancreatic progenitor cells were found to proliferate while gathering in a cluster as shown in Figure 4. This cluster was positive for DTZ staining, and stainability for insulin was detected by immunostaining. These results indicate that the pancreatic progenitor cells have differentiated into insulin-producing cells. In addition to insulin, increases in mRNA levels were actually observed for Ngn3 and glucose transporter (GLUT2) which are master transcription factors in pancreatic endocrine cells (Figure 5). Moreover, C-peptide secretion reflecting insulin secretion was also detected (Figure 5).

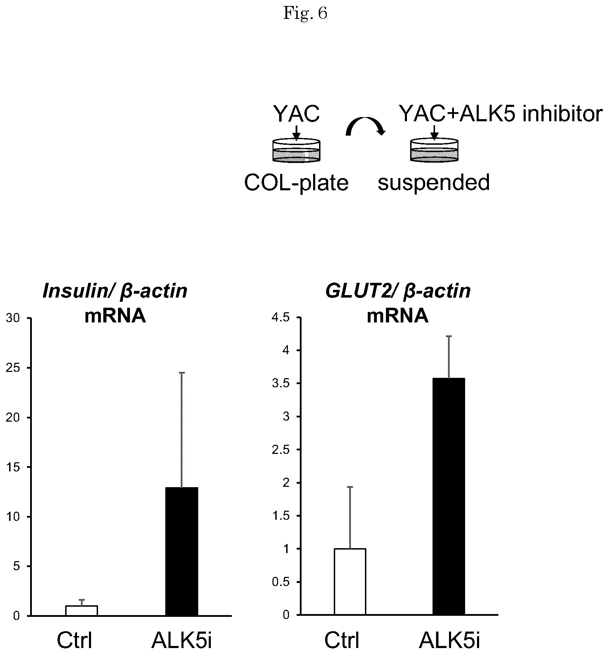

Likewise, upon floating culture for 7 days in the presence of YAC together with ALK5 inhibitor II (Enzo, ALX-270-445-M001), further improvements were possible in the mRNA expression of Insulin and GLUT2 (Figure 6).

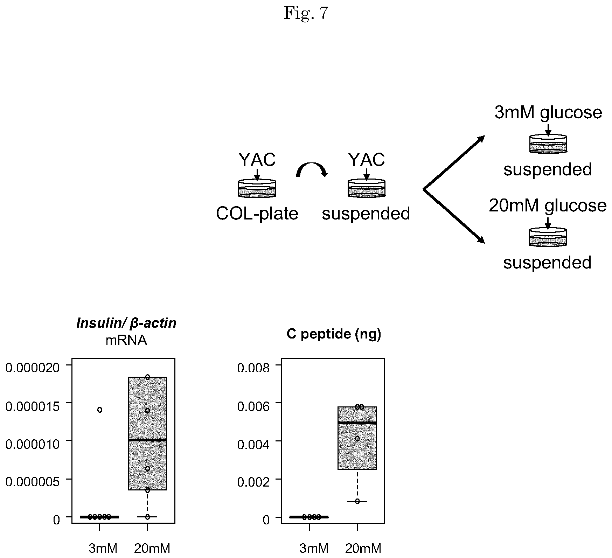

Furthermore, after induction of differentiation into pancreatic endocrine cells, a comparison was made under conditions of low (3 mM) and high (20 mM) concentrations of glucose in the medium. The high concentration of glucose was found to cause increases in Insulin mRNA and C-peptide in the medium, thus indicating that the pancreatic endocrine cells have acquired the responsiveness to glucose concentrations (Figure 7).

### 4) Experiments in animals

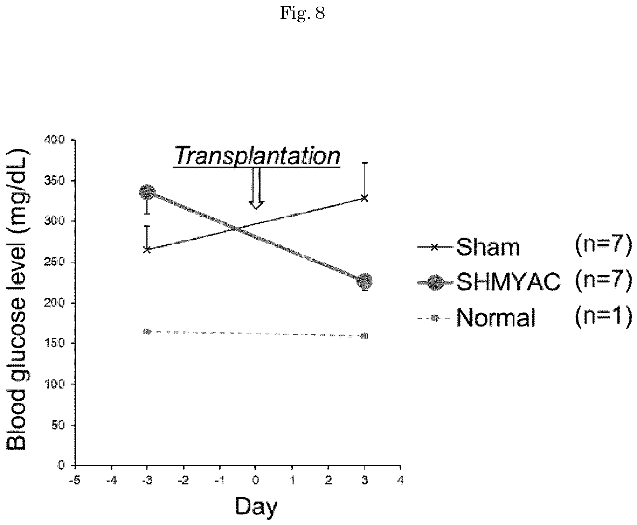

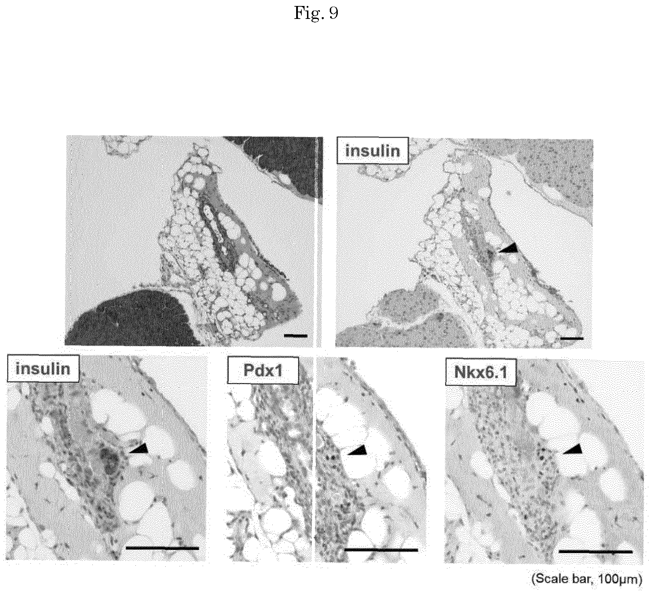

Using streptozotocin-induced diabetic model mice, pancreatic progenitor cells which had been induced to differentiate into insulin-producing cells were encapsulated within Matrigel and transplanted under the pancreatic capsule. As shown in Figure 8, at 3 days after transplantation, the mice showed improved blood glucose levels. In addition, at the transplantation site, most clusters of the transplanted cells were found to have differentiated into duct-like cells, whereas some of the cells were positive for Insulin, Pdx1 and Nkx6.1 and therefore observed to have differentiated into β cells (Figure 9).

### INDUSTRIAL APPLICABILITY

According to the present invention, pancreatic stem/progenitor cells having self-regeneration ability and differentiation potency (bipotency) into pancreatic exocrine cells can safely and rapidly be induced from pancreatic exocrine cells without genetic modification. The method of the present invention is therefore highly useful in possible applications to a drug-assessing system and pancreatic regenerative medicine.

## Claims

1. An *in vitro* method of producing pancreatic stem/progenitor cells, the method comprising contacting cells derived from mammalian pancreatic tissue with a TGFβ-receptor inhibitor, a GSK3 inhibitor and a ROCK inhibitor.

2. The *in vitro* method according to claim 1, wherein the pancreatic tissue comprises pancreatic parenchymal cells.

3. The *in vitro* method according to claim 2, wherein the pancreatic parenchymal cells comprises pancreatic exocrine cells, and optionally wherein the mammal is a human, rat, or mouse.

4. The *in vitro* method according to claim 2 or 3, wherein the contact between the cells, optionally pancreatic exocrine cells, and the TGFβ-receptor inhibitor, and optionally the GSK3 inhibitor and/or the ROCK inhibitor, is carried out by culturing the cells in the presence of the inhibitor(s).

5. The *in vitro* method according to claim 2 or 3, wherein the pancreatic tissue comprises pancreatic exocrine cells and the cells are contacted with the TGFβ-receptor inhibitor, the GSK3 inhibitor, and the ROCK inhibitor.

6. The *in vitro* method according to claim 5, wherein the TGFβ-receptor inhibitor is A-83-01 (A), the GSK3 inhibitor is CHIR99021 (C), and the ROCK inhibitor is Y-27632 (Y).

7. A method for maintaining or expanding pancreatic stem/progenitor cells comprising:
(i) producing pancreatic stem/progenitor cells by the *in vitro* method of any of claims 1-6; and
(ii) subculturing the pancreatic stem/progenitor cells on a collagen- or Matrigel-coated culture vessel in the presence of the TGFβ-receptor inhibitor, a GSK3 inhibitor and a ROCK inhibitor.

8. A method for inducing pancreatic stem/progenitor cells into cells derived from a mammalian endodermal tissue or organ or into pancreatic exocrine cells, comprising:
(i) producing pancreatic stem/progenitor cells by the *in vitro* method of any of claims 1-6; and
(ii) culturing the pancreatic stem/progenitor cells in the presence of a TGFβ-receptor inhibitor, a GSK3 inhibitor and a ROCK inhibitor.

9. An *in vitro* method for assessing the metabolism of a test compound, comprising the steps of:
(i) producing pancreatic stem/progenitor cells by the *in vitro* method of any of claims 1-6;
(ii) contacting the test compound with the pancreatic stem/progenitor cells; and
(iii) measuring the metabolism of the test compound by the cells.

10. An *in vitro* method for screening for secretion inducers of enzymes secreted from the cells comprising the steps of:
(i) producing pancreatic stem/progenitor cells by the *in vitro* method of any of claims 1-6;
(ii) contacting a test compound with the pancreatic stem/progenitor cells; and
(iii) measuring the substance(s) secreted by the cells.

11. An *in vitro* method for assessing the endodermal tissue or organ toxicity of a test compound, comprising the steps of:
(i) producing pancreatic stem/progenitor cells by the *in vitro* method of any of claims 1-6;
(ii) contacting the test compound with the pancreatic stem/progenitor cells; and
(iii) measuring the presence or the absence, or the degree of damage to the cells contacted with the test compound.

12. Use of an inducer comprising a TGFβ-receptor inhibitor in an *in vitro* method of inducing cells derived from mammalian pancreatic tissue into pancreatic stem/progenitor cells, wherein the inducer further comprises a GSK3 inhibitor and a ROCK inhibitor.

13. Use according to claim 12, wherein the pancreatic tissue comprises pancreatic parenchymal cells optionally wherein the pancreatic parenchymal cells comprise pancreatic exocrine cells, and optionally wherein the mammal is a human, a rat, or a mouse.

## Patentansprüche

1. Verfahren zur Herstellung von Pankreas-Stamm-/Vorläuferzellen *in vitro,* wobei das Verfahren das Inkontaktbringen von Zellen, die aus Pankreasgewebe von Säugetieren stammen, mit einem TGFβ-Rezeptorinhibitor, einem GSK3-Inhibitor und einem ROCK-Inhibitor umfasst.

2. Verfahren *in vitro* nach Anspruch 1, wobei das Pankreasgewebe Zellen des Pankreasparenchyms umfasst.

3. Verfahren *in vitro* nach Anspruch 2, wobei die Pankreasparenchymzellen exokrine Pankreaszellen umfassen, und wobei das Säugetier wahlweise ein Mensch, eine Ratte oder eine Maus ist.

4. Verfahren *in vitro* nach Anspruch 2 oder 3, wobei der Kontakt zwischen den Zellen, gegebenenfalls den exokrinen Zellen des Pankreas, und dem TGFß-Rezeptorinhibitor und gegebenenfalls dem GSK3-Inhibitor und/oder dem ROCK-Inhibitor durch Kultivierung der Zellen in Gegenwart des/der Inhibitors/Inhibitoren erfolgt.

5. Verfahren *in vitro* nach Anspruch 2 oder 3, wobei das Pankreasgewebe exokrine Pankreaszellen umfasst und die Zellen mit dem TGFβ-Rezeptorinhibitor, dem GSK3-Inhibitor und dem ROCK-Inhibitor in Kontakt gebracht werden.

6. Verfahren *in vitro* nach Anspruch 5, wobei der TGFβ-Rezeptorinhibitor A-83-01 (A) ist, der GSK3-Inhibitor CHIR99021 (C) ist und der ROCK-Inhibitor Y-27632 (Y) ist.

7. Verfahren zur Aufrechterhaltung oder Vermehrung von Pankreas-Stamm-/Vorläuferzellen, umfassend:
(i) Herstellen von Stamm-/Vorläuferzellen der Bauchspeicheldrüse durch das Verfahren *in vitro* nach einem der Ansprüche 1 bis 6; und
(ii) Subkultivieren der Stamm-/Vorläuferzellen der Bauchspeicheldrüse auf einem mit Kollagen oder Matrigel beschichteten Kulturgefäß in Gegenwart des TGFβ-Rezeptorinhibitors, eines GSK3-Inhibitors und eines ROCK-Inhibitors.

8. Verfahren zum Induzieren von pankreatischen Stamm-/Vorläuferzellen in Zellen, die von einem endodermalen Gewebe oder Organ eines Säugetiers stammen, oder in exokrine Pankreaszellen, umfassend:
(i) Herstellen von Stamm-/Vorläuferzellen der Bauchspeicheldrüse durch das Verfahren *in vitro* nach einem der Ansprüche 1 bis 6; und
(ii) Kultivieren der Pankreas-Stamm-/Vorläuferzellen in Gegenwart eines TGFß-Rezeptorinhibitors, eines GSK3-Inhibitors und eines ROCK-Inhibitors.

9. Verfahren *in vitro* zur Bewertung des Metabolismus einer Testverbindung, umfassend die folgenden Schritte:
(i) Herstellen von Stamm-/Vorläuferzellen der Bauchspeicheldrüse durch das Verfahren *in vitro* nach einem der Ansprüche 1 bis 6;
(ii) Inkontaktbringen der Testverbindung mit den Pankreas-Stamm-/Vorläuferzellen; und
(iii) Messen des Stoffwechsels der Testverbindung durch die Zellen.

10. Verfahren *in vitro* zum Screening von Sekretionsinduktoren von Enzymen, die aus den Zellen sekretiert werden, umfassend die folgenden Schritte:
(i) Herstellen von Stamm-/Vorläuferzellen der Bauchspeicheldrüse durch das Verfahren *in vitro* nach einem der Ansprüche 1 bis 6;
(ii) Inkontaktbringen einer Testverbindung mit den Pankreas-Stamm-/Vorläuferzellen; und
(iii) Messen der von den Zellen abgesonderten Substanz (en).

11. Verfahren *in vitro* zur Bewertung der endodermalen Gewebe- oder Organtoxizität einer Testverbindung, umfassend die folgenden Schritte:
(i) Herstellen von Stamm-/Vorläuferzellen der Bauchspeicheldrüse durch das Verfahren *in vitro* nach einem der Ansprüche 1 bis 6;
(ii) Inkontaktbringen der Testverbindung mit den Pankreas-Stamm-/Vorläuferzellen; und
(iii) Messen der Anwesenheit oder Abwesenheit oder des Ausmaßes der Schädigung der Zellen, die mit der Testverbindung in Kontakt gekommen sind.

12. Verwendung eines Induktors, umfassend einen TGFβ-Rezeptorinhibitor, in einem Verfahren *in vitro* zur Induktion von aus Säugetierpankreasgewebe stammenden Zellen zu Pankreasstamm-/Vorläuferzellen, wobei der Induktor ferner einen GSK3-Inhibitor und einen ROCK-Inhibitor umfasst.

13. Verwendung nach Anspruch 12, wobei das Pankreasgewebe Pankreasparenchymzellen umfasst, wobei die Pankreasparenchymzellen optional Pankreasexokrinzellen umfassen und wobei das Säugetier optional ein Mensch, eine Ratte oder eine Maus ist.

## Revendications

1. Procédé *in vitro* de production de cellules souches/progénitrices pancréatiques, le procédé consistant à mettre en contact des cellules dérivées d'un tissu pancréatique de mammifère avec un inhibiteur du récepteur TGFβ, un inhibiteur de GSK3 et un inhibiteur de ROCK.

2. Procédé *in vitro* selon la revendication 1, dans laquel le tissu pancréatique comprend des cellules parenchymateuses du pancréas.

3. Procédé *in vitro* selon la revendication 2, dans lequel les cellules parenchymateuses du pancréas comprennent des cellules exocrines du pancréas, et éventuellement dans lequel le mammifère est un humain, un rat ou une souris.

4. Procédé *in vitro* selon la revendication 2 ou 3, dans lequel le contact entre les cellules, éventuellement les cellules exocrines pancréatiques, et l'inhibiteur du récepteur TGFβ, et éventuellement l'inhibiteur GSK3 et/ou l'inhibiteur ROCK, est effectué en cultivant les cellules en présence du ou des inhibiteurs.

5. Procédé *in vitro* selon la revendication 2 ou 3, dans lequel le tissu pancréatique comprend des cellules exocrines pancréatiques et les cellules sont mises en contact avec l'inhibiteur du récepteur TGFβ, l'inhibiteur GSK3 et l'inhibiteur ROCK.

6. Procédé *in vitro* selon la revendication 5, dans lequel l'inhibiteur du récepteur TGFβ est A-83-01 (A), l'inhibiteur GSK3 est CHIR99021 (C), et l'inhibiteur ROCK est Y-27632 (Y).

7. Procédé de maintien ou d'expansion de cellules souches/progénitrices pancréatiques comprenant :
(i) produire des cellules souches/progénitrices pancréatiques par le procédé *in vitro* selon l'une quelconque des revendications 1 à 6 ; et
(ii) sous-cultiver des cellules souches/progénitrices pancréatiques dans un récipient de culture recouvert de collagène ou de Matrigel en présence de l'inhibiteur du récepteur TGFβ, d'un inhibiteur de GSK3 et d'un inhibiteur de ROCK.

8. Procédé d'induction de cellules souches/progénitrices pancréatiques en cellules dérivées d'un tissu ou d'un organe endodermique de mammifère ou en cellules exocrines pancréatiques, comprenant :
(i) produire des cellules souches/progénitrices pancréatiques par le procédé *in vitro* selon l'une quelconque des revendications 1 à 6 ; et
(ii) cultiver des cellules souches/progénitrices pancréatiques en présence d'un inhibiteur du récepteur TGFβ, d'un inhibiteur de GSK3 et d'un inhibiteur de ROCK.

9. Procédé *in vitro* pour évaluer le métabolisme d'un composé test, comprenant les étapes suivantes :
(i) produire des cellules souches/progénitrices pancréatiques par le procédé *in vitro* selon l'une quelconque des revendications 1 à 6 ;
(ii) mettre en contact le composé test avec les cellules souches/progénitrices pancréatiques ; et
(iii) mesurer le métabolisme du composé test par les cellules.

10. Procédé *in vitro* de criblage d'inducteurs de la sécrétion d'enzymes sécrétées par les cellules, comprenant les étapes suivantes :
(i) produire des cellules souches/progénitrices pancréatiques par le procédé *in vitro* selon l'une quelconque des revendications 1 à 6 ;
(ii) mettre en contact d'un composé test avec les cellules souches/progénitrices pancréatiques ; et
(iii) mesurer la ou les substances sécrétées par les cellules.

11. Procédé *in vitro* d'évaluation de la toxicité d'un composé test pour les tissus ou organes endodermiques, comprenant les étapes suivantes :
(i) produire des cellules souches/progénitrices pancréatiques par le procédé *in vitro* selon l'une quelconque des revendications 1 à 6 ;
(ii) mettre en contact le composé test avec les cellules souches/progénitrices pancréatiques ; et
(iii) mesurer la présence, l'absence ou le degré d'atteinte des cellules mises en contact avec le composé test.

12. Utilisation d'un inducteur comprenant un inhibiteur du récepteur TGFβ dans un procédé *in vitro* d'induction de cellules dérivées de tissus pancréatiques de mammifère en cellules souches/progénitrices pancréatiques, dans lequel l'inducteur comprend en outre un inhibiteur de GSK3 et un inhibiteur de ROCK.

13. Utilisation selon la revendication 12, dans laquelle le tissu pancréatique comprend des cellules parenchymateuses pancréatiques, éventuellement dans laquelle les cellules parenchymateuses pancréatiques comprennent des cellules exocrines pancréatiques, et éventuellement dans laquelle le mammifère est un humain, un rat ou une souris.
